# EUROPEAN PATENT APPLICATION

(11) **EP 2 305 373 A1**
(43) Date of publication of application: **06.04.2011**
(21) Application number: 09800416.1
(22) Date of filing: 22.07.2009
(51) Int. Cl.: B01J 13/04, A23L 1/00, A61K 9/50, A61K 47/22, A61K 47/36

(54) **MICROCAPSULE, PROCESS FOR PRODUCTION THEREOF, AND FOOD OR BEVERAGE CONTAINING MICROCAPSULE**

(30) Priority: 24.07.2008 JP 2008191360
(71) Applicant: Niigata University, Niigata-shi Niigata 950-2181 (JP); Sapporo Breweries Limited, Tokyo 150-8522 (JP)
(72) Inventor: TANAKA Masato, Niigata-shi Niigata 950-2181 (JP); TSUCHIMOTO Norihiko, Tokyo 150-8522 (JP)
(74) Representative: Vossius & Partner
(86) International application number: PCT/JP2009/063124
(87) International publication number: WO 2010/010902

(57) **Abstract**

The method of producing microcapsules 100 comprises a primary dispersion step in which a water-soluble substance and a fat-soluble substance are mixed to obtain a primary dispersion liquid having primary dispersion particles 10 composed of the water-soluble substance dispersed in the fat-soluble substance, a secondary dispersion step in which the primary dispersion liquid and a sodium alginate aqueous solution are mixed to obtain a secondary dispersion liquid having secondary dispersion particles 20 composed of the primary dispersion liquid dispersed in the sodium alginate aqueous solution, and a spraying step in which the secondary dispersion liquid is sprayed for contact with a calcium ion-containing solution 9 to form a calcium alginate gel 30 and to obtain microcapsules 100 having the secondary dispersion particles 20 dispersed in the calcium alginate gel 30.

## Description

### Technical Field

The present invention relates to microcapsules and to a method of producing thereof, and to a food and beverage containing the microcapsules.

### Background Art

Microcapsules have a covering layer as a shell substance formed around a functional encapsulated substance serving as the core substance, to protect it. In recent years, microcapsules have come to be used in a variety of fields, by combining encapsulated core substances and shell substances that cover the core substances. Production of microcapsules is being studied in particular for use in foods, medicines and the like (see Patent literatures 1-4).

Microcapsules are known to have multilayer structures such as W/O or W/O/W. Microcapsules with W/O/W three-layer structures are obtained by drying in water phase method (see Patent literature 5, for example) or by methods using multiple nozzles (see Patent literatures 6 and 7, for example).

### Citation List

### Patent Literature

[Patent literature 1] Japanese Unexamined Patent Application Publication HEI No. 05-049899
[Patent literature 2] Japanese Patent Public Inspection No. 2002-511796
[Patent literature 3] Japanese Unexamined Patent Application Publication HEI No. 05-049433
[Patent literature 4] Japanese Unexamined Patent Application Publication HEI No. 06-2543 82
[Patent literature 5] Japanese Unexamined Patent Application Publication HEI No. 05-031352
[Patent literature 6] Japanese Unexamined Patent Application Publication HEI No. 06-055060
[Patent literature 7] Japanese Unexamined Patent Application Publication HEI No. 08-010313

### Summary of Invention

### Technical Problem

Microcapsules used for foods or medicines are required to be microcapsules with small particle sizes, from the viewpoint of improving the feel of the microcapsules in the mouth/throat and stomach. With the microcapsules having W/O/W three-layer structures described in Patent literatures 5-7, however, it has not been possible to produce microcapsules with small particle sizes.

It is therefore an object of the present invention to provide a method of producing microcapsules by which the microcapsules with small particle sizes can be obtained, microcapsules obtainable by the production method, and foods and beverages containing the microcapsules.

### Solution to Problem

The invention provides a method of producing microcapsules that comprises a primary dispersion step of mixing a water-soluble substance and a fat-soluble substance to obtain a primary dispersion having the water-soluble substance dispersed in the fat-soluble substance, a secondary dispersion step of mixing the primary dispersion and a sodium alginate aqueous solution to obtain a secondary dispersion having the primary dispersion dispersed in the sodium alginate aqueous solution, and a spraying step of spraying the secondary dispersion for contact with a calcium ion-containing solution to form a calcium alginate gel and to obtain microcapsules having the primary dispersion dispersed in the calcium alginate gel.

The invention further provides a method of producing microcapsules that comprises a step of obtaining microcapsules having a primary dispersion dispersed in a calcium alginate gel by contacting droplets of a secondary dispersion liquid with a calcium ion-containing solution to form the calcium alginate gel, wherein the primary dispersion has a water-soluble substance dispersed in a fat-soluble substance, and the secondary dispersion liquid comprises the primary dispersion dispersed in a sodium alginate aqueous solution.

According to the invention, the microcapsules are produced by spraying a secondary dispersion in which a primary dispersion is dispersed in a sodium alginate aqueous solution, for contact with a calcium ion-containing solution, or by contacting droplets of a secondary dispersion liquid in which a primary dispersion is dispersed in a sodium alginate aqueous solution, with a calcium ion-containing solution, thus allowing reduction in the particle sizes of obtained microcapsules.

The water-soluble substance that is dispersed in the primary dispersion may be a water-soluble substance dispersing a fat-soluble substance that is the same or different from the aforementioned fat-soluble substance (a water-soluble substance that is the same or different from the aforementioned water-soluble substance may be dispersed in the fat-soluble substance, or dispersion of either a fat-soluble substance or a water-soluble substance in the other may be repeated several times more.)

The viscosity of the sodium alginate aqueous solution at 25°C is preferably 5-2000 mPa·s. This will result in even smaller particle sizes of the microcapsules.

In the method of producing microcapsules according to the invention, the calcium ion-containing solution is preferably a calcium chloride aqueous solution, a calcium lactate aqueous solution or a calcium sulfate aqueous solution. This will allow the particles composed of the secondary dispersion liquid to be instantaneously encapsulated in the calcium alginate gel by spraying, to allow microcapsules with even smaller particle sizes to be obtained.

The invention also provides microcapsules obtainable by the production method described above. Such microcapsules have small particle sizes.

The invention further provides microcapsules comprising a calcium alginate gel and having a mean particle size of less than 200 µm, wherein a fat-soluble substance is dispersed in the calcium alginate gel and a water-soluble substance is dispersed in the fat-soluble substance. Such microcapsules have small particle sizes and can therefore be added to various foods and beverages without impairing the feel of the microcapsules in the mouth/throat.

The water-soluble substance may be a water-soluble substance dispersing a fat-soluble substance that is the same or different from the aforementioned fat-soluble substance (a water-soluble substance that is the same or different from the aforementioned water-soluble substance may be dispersed in the fat-soluble substance, or dispersion of either a fat-soluble substance or a water-soluble substance in the other may be repeated several times more.).

The microcapsules preferably have a degree of deformation of less than 1.8. Since such microcapsules are nearly spherical and have excellent durability, leakage of water-soluble substances and fat-soluble substances into the foods and beverages can be inhibited, thus preventing reduction in the quality of the foods and beverages.

The invention further provides foods and beverages containing the microcapsules of the invention. Since such foods and beverages contain microcapsules with small particle sizes, they have excellent feel of the microcapsules in the mouth/throat and stomach.

### Advantageous Effects of Invention

According to the invention, it is possible to provide a method of producing microcapsules by which the microcapsules with small particle sizes can be obtained, microcapsules obtainable by the production method, and foods and beverages containing the microcapsules.

### Brief Description of Drawings

Fig. 1 is a schematic view showing a method of producing microcapsules according to the invention.
Fig. 2 is a schematic view showing a method of producing microcapsules according to the invention.
Fig. 3 is a schematic view showing a method of producing microcapsules according to the invention.
Fig. 4 is a schematic view showing microcapsules formed by the production method according to an embodiment of the invention.
Fig. 5 is an optical microscope photograph of the microcapsules formed in Example 1.
Fig. 6 is an optical microscope photograph of the microcapsules formed in Example 4.
Fig. 7 is an optical microscope photograph of the microcapsules formed in Example 5.
Fig. 8 is an optical microscope photograph of the microcapsules formed in Example 13.
Fig. 9 is an optical microscope photograph of the microcapsules formed in Example 19.

### Description of Embodiments

Preferred embodiments of the invention will now be explained in detail, with reference to the accompanying drawings as necessary. Throughout the drawings, corresponding elements will be referred to by like reference numerals and will be explained only once. Unless otherwise specified, the vertical and horizontal positional relationships are based on the positional relationships in the drawings. Also, the dimensional proportions depicted in the drawings are not necessarily limitative.

### (Method of producing microcapsules)

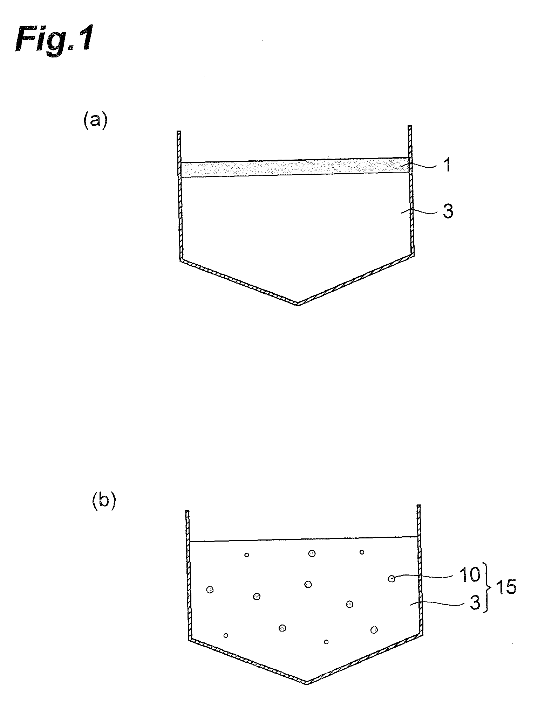

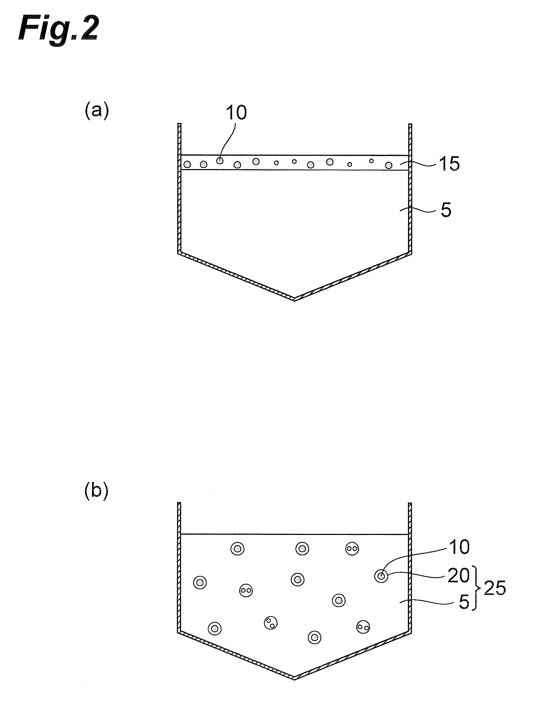

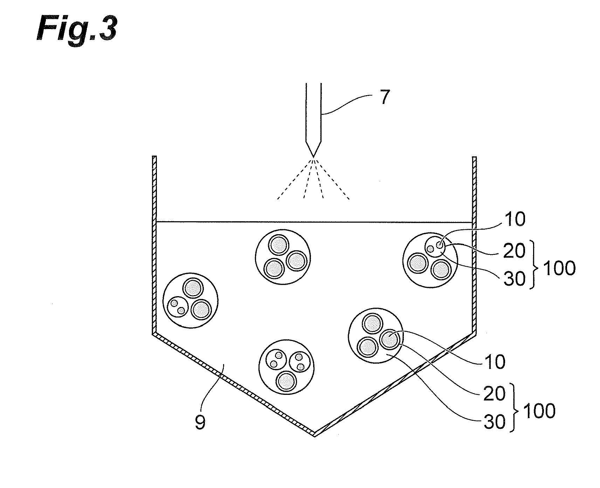

Figs. 1 to 3 are schematic views showing a method of producing microcapsules according to an embodiment of the invention. The method of producing microcapsules according to this embodiment comprises a primary dispersion step in which a water-soluble substance 1 and a fat-soluble substance 3 are mixed to obtain a primary dispersion liquid (a primary dispersion) 15 having primary dispersion particles 10 composed of the water-soluble substance 1 dispersed in the fat-soluble substance 3 (Fig. 1), a secondary dispersion step in which the primary dispersion liquid 15 and a sodium alginate aqueous solution 5 are mixed to obtain a secondary dispersion liquid (a secondary dispersion) 25 having secondary dispersion particles 20 composed of the primary dispersion liquid 15 dispersed in the sodium alginate aqueous solution 5 (Fig. 2), and a spraying step in which the secondary dispersion liquid 25 is sprayed for contact with a calcium ion-containing solution 9 to form a calcium alginate gel 30 and to obtain microcapsules 100 having the secondary dispersion particles 20 dispersed in the calcium alginate gel 30 (Fig. 3).

Each step of the method of producing microcapsules according to this embodiment will now be explained in detail with reference to Figs. 1 to 3.

### (Primary dispersion step)

First, as shown in Fig. 1(a), an aqueous layer comprising a water-soluble substance (hereunder referred to simply as "water-soluble substance") 1 and an oil layer comprising a fat-soluble substance (hereunder referred to simply as "fat-soluble substance") 3 are prepared, and then as shown in Fig. 1(b), the water-soluble substance 1 and fat-soluble substance 3 are mixed together to disperse the primary dispersion particles 10 composed of the water-soluble substance 1 in the fat-soluble substance 3 (hereunder also referred to as "primary dispersion"). The method of conducting the primary dispersion may be a method known in the prior art, and for example, a homomixer or homogenizer may be used for mixing and dispersion.

The water-soluble substance 1 is not particularly restricted so long as it is suitable for use in foods and beverages, and examples include water-soluble bioactive substances, starches and bittering agents. Water-soluble bioactive substances include water-soluble vitamins such as ascorbic acid, thiamine, riboflavin, niacin, pantothenic acid, biotin, vitamin B6 (pyridoxine, pyridoxal, pyridoxamine and the like), folic acid and cyanocobalamin, water-soluble dietary fiber (pectin enzyme hydrolyzed guar gum, agarose, glucomannan, polydextrose and the like), dextrin, caffeine, naringin, amino acids, amino acid derivatives, water-soluble peptides, water-soluble proteins and water-soluble polyphenols. The water-soluble substance 1 may be used as a single type or a combination of two or more.

The viscosity of the water-soluble substance 1 at 25°C is preferably not greater than 10000 mPa·s and more preferably not greater than 5000 mPa·s. If the viscosity of the water-soluble substance 1 exceeds 10000 mPa·s, dispersion (emulsification) will be more difficult and the particle sizes of the primary dispersion particles 10 will tend to be increased.

The fat-soluble substance 3 is not particularly restricted so long as it is one that is used for foods and beverages, and it may be a fat-soluble bioactive substance, examples of which include fat-soluble vitamins such as vitamin A compounds, vitamin D compounds, vitamin E compounds and vitamin K compounds, coenzyme Q compounds such as ubiquinone, or astaxanthin, zeaxanthin, fucoxanthin, β-carotene, DHA, EPA and edible fats and oils (corn oil, rapeseed oil, soybean oil and the like). Vitamin A compounds include retinol, retinoic acid, retinoids and carotenes, vitamin D compounds include cholecalciferol and ergocalciferol, vitamin E compounds include tocopherol, tocopherol acetate, tocopherol succinate, tocopherol nicotinate and tocotrienol, and vitamin K compounds include phytonadione and menatetrenone. The fat-soluble substance 3 may be used as a single type or a combination of two or more.

The viscosity of the fat-soluble substance 3 at 25°C is preferably 10-10000 mPa·s and more preferably 20-5000 mPa·s. If the viscosity of the fat-soluble substance 3 is greater than 10000 mPa·s, dispersion (emulsification) will tend to become difficult, and if it is less than 10 mPa·s, the dispersed (emulsified) particles will tend to form masses, thus increasing the particle sizes of the primary dispersion particles 10.

From the viewpoint of satisfactorily dispersing the water-soluble substance 1, the mixing proportion of the water-soluble substance 1 is preferably not greater than 100 parts by mass, more preferably not greater than 50 parts by mass, even more preferably not greater than 40 parts by mass, and most preferably not greater than 30 parts by mass, with respect to 100 parts by mass of the fat-soluble substance 3. From the viewpoint of improving the yield of the microcapsules, the lower limit for the mixing proportion of the water-soluble substance 1 is preferably about 10 parts by mass.

Also, from the viewpoint of satisfactorily dispersing the water-soluble substance 1, the mixing proportion of the water-soluble substance 1 is preferably not greater than 100 parts by volume, more preferably not greater than 50 parts by volume, even more preferably not greater than 40 parts by volume and most preferably not greater than 30 parts by volume, with respect to 100 parts by volume of the fat-soluble substance 3. From the viewpoint of improving the yield of the microcapsules, the lower limit for the mixing proportion of the water-soluble substance 1 is preferably about 10 parts by volume.

In the primary dispersion step, an emulsifier may be added for emulsification during mixture of the water-soluble substance 1 and fat-soluble substance 3, as necessary, thus allowing formation of a more stable primary dispersion liquid 15. The emulsifier is not particularly restricted so long as it is one used for medicines, foods and beverages, and examples include glycerin fatty acid esters, glycerin acetate fatty acid esters, glycerin lactate fatty acid esters, glycerin succinate fatty acid esters, glycerin diacetyltartrate fatty acid esters, sorbitan fatty acid esters, sucrose fatty acid esters, sucrose acetate isobutyric acid ester, polyglycerin fatty acid esters, polyglycerin-condensed ricinoleic acid ester, propyleneglycol fatty acid esters, calcium stearoyl lactate, sodium stearoyl lactate, polyoxyethylenesorbitan monostearate, polyoxyethylenesorbitan monoglyceride and lecithin. The amount of emulsifier added is preferably about 0.01-15 parts by mass with respect to 100 parts by mass of the fat-soluble substance 3.

In the primary dispersion step, the primary dispersion liquid 15 may be stirred by a method with even greater speed and pressure for microdispersion of the primary dispersion particles 10 in the fat-soluble substance 3. The method for microdispersion of the primary dispersion particles 10 is preferably one in which a high shear force is applied, and it may be a method of stirring using, for example, a high-pressure homogenizer, nanomizer, homomixer, colloid mill, Disper mixer mill or static mixer.

This will allow a primary dispersion liquid 15 to be obtained having primary dispersion particles 10 composed of the water-soluble substance 1, dispersed in the fat-soluble substance 3.

### (Secondary dispersion step)

First, as shown in Fig. 2(a), there are prepared a primary dispersion liquid 15 comprising primary dispersion particles 10 prepared as described above, and a sodium alginate aqueous solution 5. Next, as shown in Fig. 2(b), the primary dispersion liquid 15 and sodium alginate aqueous solution 5 are mixed to disperse secondary dispersion particles 20 encapsulating one or multiple primary dispersion particles 10 in the sodium alginate aqueous solution 5, to obtain a secondary dispersion liquid 25 (hereunder also referred to as "secondary dispersion"). The method of conducting the secondary dispersion may be a method known in the prior art, and for example, a homomixer or homogenizer may be used for mixing and dispersion.

The mean particle size of the secondary dispersion particles 20 is preferably not greater than 20 µm, more preferably not greater than 10 µm and even more preferably not greater than 5 µm. If the mean particle size of the secondary dispersion particles 20 is greater than 20 µm, it will be difficult to reduce the microcapsule particle sizes, concavities and convexities will form in the microcapsules and spherical shapes will not be easily obtained. The mean particle size of the secondary dispersion particles 20 can be measured using a laser diffraction/scattering particle size distribution meter, and it is referred to as the volume-average particle size.

From the viewpoint of satisfactorily dispersing the secondary dispersion particles 20, the mixing proportion of the primary dispersion liquid 15 is preferably not greater than 100 parts by mass, more preferably not greater than 50 parts by mass and even more preferably not greater than 40 parts by mass, with respect to 100 parts by mass of the sodium alginate aqueous solution 5. The mixing proportion of the primary dispersion liquid 15 is most preferably not greater than 20 parts by mass from the viewpoint of further reducing the mean particle size of the microcapsules, and improving the degree of deformation. From the viewpoint of improving the yield of the microcapsules, the lower limit for the mixing proportion of the primary dispersion liquid 15 is preferably about 5 parts by mass.

Also, from the viewpoint of satisfactorily dispersing the secondary dispersion particles 20, the mixing proportion of the primary dispersion liquid 15 is preferably not greater than 100 parts by volume, more preferably not greater than 50 parts by volume and even more preferably not greater than 40 parts by volume, with respect to 100 parts by volume of the sodium alginate aqueous solution 5. The mixing proportion of the primary dispersion liquid 15 is most preferably not greater than 20 parts by volume from the viewpoint of further reducing the mean particle size of the microcapsules, and improving the degree of deformation. From the viewpoint of improving the yield of the microcapsules, the lower limit for the mixing proportion of the primary dispersion liquid 15 is preferably about 5 parts by volume.

The concentration of the sodium alginate aqueous solution 5 is preferably 0.1-5.0 mass%, more preferably 0.5-3.0 mass% and even more preferably 0.5-2.0 mass%. If the concentration of the sodium alginate aqueous solution 5 is less than 0.1 mass%, gelling of the calcium alginate in the spraying step described hereunder will tend to be more difficult, and if it exceeds 5.0 mass%, the secondary dispersion liquid 25 will not readily flow in the supply passage during the spraying step, and it will not easily be sprayed by the nozzle.

The viscosity of the sodium alginate aqueous solution 5 at 25°C is preferably 5-2000 mPa·s, more preferably 10-500 mPa·s and even more preferably 15-100 mPa·s. If the viscosity of the sodium alginate aqueous solution 5 is less than 5 mPa·s, the durability of the microcapsules will tend to be lowered, and if it is greater than 2000 mPa·s, the particle sizes of the microcapsules will tend to be increased.

In the secondary dispersion step, an emulsifier may be added for emulsification during mixture of the secondary dispersion liquid 25 and sodium alginate aqueous solution 5, as necessary, thus allowing formation of a more stable secondary dispersion liquid 25. The emulsifier is not particularly restricted so long as it is one used for medicines, foods and beverages, and examples include glycerin fatty acid esters, glycerin acetate fatty acid esters, glycerin lactate fatty acid esters, glycerin succinate fatty acid esters, glycerin diacetyltartrate fatty acid esters, sorbitan fatty acid esters, sucrose fatty acid esters, sucrose acetate isobutyric acid ester, polyglycerin fatty acid esters, polyglycerin-condensed ricinoleic acid ester, propyleneglycol fatty acid esters, calcium stearoyl lactate, sodium stearoyl lactate, polyoxyethylene sorbitan monostearate, polyoxyethylene sorbitan monoglyceride and lecithin. The amount of emulsifier added is preferably about 0.1-5 parts by mass with respect to the sodium alginate aqueous solution.

In the secondary dispersion step, the secondary dispersion liquid 25 may be stirred by a method with even greater speed and pressure for microdispersion of the secondary dispersion particles 20 in the sodium alginate aqueous solution 5. The method of microdispersion of the secondary dispersion particles 20 is preferably one in which a high shear force is applied, and it may be a method of stirring using, for example, a high-pressure homogenizer, nanomizer, homomixer, colloid mill, Disper mixer mill or static mixer.

This will allow a secondary dispersion liquid 25 to be obtained having the primary dispersion liquid 15, comprising the water-soluble substance 1 dispersed in the fat-soluble substance 3, dispersed in the sodium alginate aqueous solution 5 as secondary dispersion particles 20.

### (Spraying step)

Next, the secondary dispersion liquid 25 is sprayed as a mist into the calcium ion-containing solution 9 through a nozzle 7, as shown in Fig. 3, to form microcapsules 100 having the secondary dispersion particles 20 encapsulated in the calcium alginate gel 30. By spraying the secondary dispersion liquid 25 in this manner, the droplets of the secondary dispersion liquid 25 are contacted with the calcium ion-containing solution 9, and microcapsules 100 are obtained having the droplets of the secondary dispersion liquid 25 encapsulated by the calcium alginate gel 30.

In other words, the calcium ion-containing solution 9 functions as a gelling agent (coagulant), and when the secondary dispersion liquid 25 is sprayed into the calcium ion-containing solution 9, the sodium alginate on the droplet surfaces of the sprayed secondary dispersion liquid 25 reacts with the calcium ion, forming a gel of insoluble calcium alginate. As a result, the secondary dispersion particles 20 are encapsulated in the calcium alginate gel 30, forming microcapsules 100.

The calcium ion-containing solution 9 is preferably a calcium chloride aqueous solution, calcium lactate aqueous solution or calcium sulfate aqueous solution, from the viewpoint of instantaneous gelling, and it is more preferably a calcium chloride aqueous solution from the viewpoint of easier release of calcium ion.

The calcium ion concentration of the calcium ion-containing solution 9 is preferably 0.5-20 mass%, more preferably 0.5-10 mass% and even more preferably 1-10 mass%. If the calcium ion concentration is less than 0.5 mass%, it will tend to be more difficult to obtain a gel, and if it exceeds 20 mass%, the cost will be increased and a longer time will tend to be necessary for the washing step described below.

The discharge slit diameter of the nozzle 7 is preferably not greater than 1.7 mm, more preferably not greater than 1.2 mm and even more preferably not greater than 1.1 mm. If the discharge slit diameter is greater than 1.7 mm, the particle size of the microcapsules will tend to be increased. The nozzle 7 may have a single discharge slit, or more than one.

The spraying gas pressure through the nozzle 7 during spraying of the secondary dispersion liquid 25 is preferably 0.1-1.0 MPa and more preferably 0.3-0.5 MPa. If the pressure is less than 0.1 MPa, the particle sizes of the microcapsules will tend to increase, and if it is greater than 1.0 MPa, concavities and convexities will be produced in the microcapsules and the degree of deformation of the microcapsules will tend to be increased.

The liquid conveyance speed of the secondary dispersion liquid 25 through the nozzle 7 is preferably 0.1-2.0 mL/min and more preferably 0.25-1.0 mL/min. If the liquid conveyance speed is less than 0.1 mL/min, the production efficiency will tend to be lower, and if it is greater than 2.0 mL/min, the particle sizes of the microcapsules will tend to be larger.

### (Washing step)

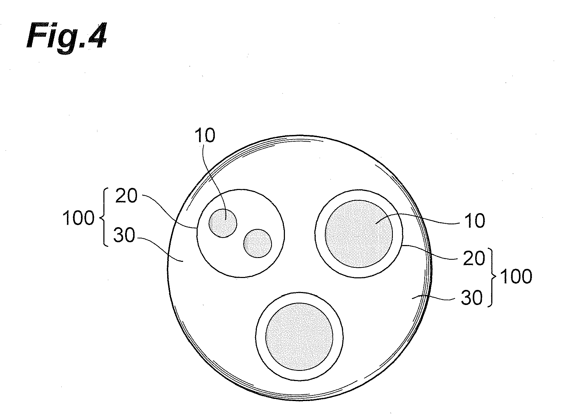

Next, the microcapsules 100 are filtered and collected, and appropriate washing treatment and classification are carried out depending on the purpose of use, for isolation as W/O/W trilayer microcapsules. Fig. 4 is a schematic view showing microcapsules 100 formed by the production method according to an embodiment of the invention. This embodiment allows the secondary dispersion particles 20 encapsulating the primary dispersion particles 10 to be encapsulated in the calcium alginate gel 30, to obtain nearly spherical microcapsules 100 with small particle sizes. That is, the microcapsules 100 have a three-layer structure comprising a layer composed of the water-soluble substance 1, a layer composed of the fat-soluble substance 3 and a layer composed of the calcium alginate gel 30, and the secondary dispersion particles (complex emulsion) 20 has the water-soluble substance 1 dispersed in the fat-soluble substance 3.

The mean particle size of the microcapsules 100 is preferably less than 200 µm, more preferably not greater than 100 µm and more preferably not greater than 50 µm. If the mean particle size of the microcapsules 100 is 200 µm or greater, and they are added to foods or beverages, the foods or beverages will tend to have an inferior feel of the microcapsules in the mouth/throat and stomach. The mean particle size of the microcapsules 100 can be measured using a laser diffraction/scattering particle size distribution meter, and it is referred to as the volume-average particle size.

The degree of deformation of the microcapsules 100 is preferably less than 1.8, more preferably less than 1.6 and even more preferably less than 1.4. With the degree of deformation of 1.8 or greater, the durability of the microcapsules will tend to be lower. The degree of deformation is the value determined by measuring the long diameter (the longest diameter of the microcapsules) and the short diameter (the shortest diameter of the microcapsules) from a photograph of the microcapsules taken with an optical microscope, and dividing the long diameter by the short diameter. That is, the degree of deformation approaching 1.00 is more nearly spherical.

The contained ratio of the water-soluble substance 1 encapsulated in the microcapsules is preferably 0.1% or greater and more preferably 0.5% or greater, with respect to the total microcapsules. The contained ratio can be obtained in the following manner. First, the microcapsules are dried under prescribed drying conditions, and after adding ethanol, they are crushed. The ethanol solution containing the crushed microcapsules are centrifuged, and then the absorbance of the supernatant is measured, and the content (mass proportion) of the water-soluble substance 1 with the moisture in the dry microcapsules removed is calculated, to obtain the contained ratio.

The contained ratio of the fat-soluble substance 3 encapsulated in the microcapsules is preferably 50% or greater and more preferably 60% or greater, with respect to the total microcapsules. The contained ratio is obtained by calculating the content (mass proportion) of the fat-soluble substance 3 in the dry microcapsules by the same method as for the contained ratio of the water-soluble substance 1.

The microcapsules 100 of this embodiment can be used in medicines, functional foods and beverages, or food and beverage additives, by appropriately varying the encapsulated water-soluble substance 1 and fat-soluble substance 3. They are particularly suitable for addition to foods and beverages because they have small particle sizes and are spherical. The foods and beverages containing the microcapsules 100 therefore have sufficiently excellent feel of the microcapsules in the mouth/throat and stomach. Since such microcapsules 100 are nearly spherical and have excellent durability, leakage of the water-soluble substance 1 and the fat-soluble substance 3 into the foods and beverages can be inhibited, thus preventing reduction in the quality of the foods and beverages.

The present invention is not in any way limited to the preferred embodiment described above.

The microcapsules of this embodiment are microcapsules comprising a calcium alginate gel, wherein a fat-soluble substance is dispersed in the calcium alginate gel and a water-soluble substance is dispersed in the fat-soluble substance, but there is no limitation to a three-layer structure. For example, they may be microcapsules having a structure with four or more layers, such as microcapsules wherein the water-soluble substance is a water-soluble substance dispersing a fat-soluble substance that is the same or different from the aforementioned fat-soluble substance (a water-soluble substance that is the same or different from the aforementioned water-soluble substance may be dispersed in the fat-soluble substance, or dispersion of either a fat-soluble substance or a water-soluble substance in the other may be repeated several times more.).

Microcapsules having a structure with four or more layers may have the water-soluble substance dispersed in the primary dispersion liquid, as a water-soluble substance dispersing a fat-soluble substance that is the same or different from the aforementioned fat-soluble substance (a water-soluble substance that is the same or different from the aforementioned water-soluble substance may be dispersed in the fat-soluble substance, or dispersion of either a fat-soluble substance or a water-soluble substance in the other may be repeated several times more).

Microcapsules with a four-layer structure can be obtained by the following steps, for example. First, in the primary dispersion step, the water-soluble substance and fat-soluble substance are mixed together to prepare a dispersion liquid comprising the fat-soluble substance dispersed in the water-soluble substance, and the dispersion liquid is further dispersed in the fat-soluble substance to prepare a primary dispersion liquid. Next, in the secondary dispersion step, the primary dispersion liquid and a sodium alginate aqueous solution are mixed together to prepare a secondary dispersion liquid having the primary dispersion liquid dispersed in the sodium alginate aqueous solution. The same spraying step and washing step may be carried out as in the embodiment described above, to obtain microcapsules with a four-layer structure.

Microcapsules having a structure with five or more layers can also be obtained by, in the primary dispersion step, carrying out several repetitions of a step in which a fat-soluble substance having a water-soluble substance dispersed therein is further dispersed in a water-soluble substance, or a step in which a water-soluble substance having a fat-soluble substance dispersed therein is further dispersed in a fat-soluble substance, to produce a primary dispersion liquid, and subjecting the dispersion liquid to the same secondary dispersion step, spraying step and washing step as in the embodiment described above.

### Examples

The present invention will now be explained in detail by examples, with the understanding that the invention is not limited to the examples.

### (Example 1)

Polyphenol powder (grape seed extract OPC30 by Samplite) was dissolved in distilled water to prepare a 20 mass% polyphenol aqueous solution. Next, 1.6 g of an emulsifier (trade name: "POEM PR-100", product of Riken Vitamin Co., Ltd.) was dissolved in 14.4 g of vitamin E (product of Wako Pure Chemical Industries, Ltd.), and then 4.0 g of a polyphenol aqueous solution was further added to prepare a solution, which was subjected to primary dispersion (emulsification) using a homomixer (trade name: "BM-2", product of Nippon Seiki Co., Ltd.) under conditions of 12000 rpm, 5 minutes, 60°C, to prepare a W/O dispersion liquid.

There was also prepared 176.4 g of a 1 mass% sodium alginate aqueous solution with a viscosity of 80 mPa·s, having sodium alginate (product of Wako Pure Chemical Industries, Ltd.) dissolved in distilled water, and then 3.6 g of an emulsifier (trade name: "ML-750", product of Sakamoto Yakuhin Kogyo Co., Ltd.) and 20 g of the W/O dispersion liquid were added, and the mixture was subjected to secondary dispersion (emulsification) using a homomixer (trade name: "BM-2", product of Nippon Seiki Co., Ltd.) under conditions of 8000 rpm, 5 minutes, 60°C, to prepare a W/O/W dispersion liquid containing secondary dispersion particles with a volume-average particle size of 3 µm.

The W/O/W dispersion liquid was then sprayed through a spray nozzle (trade name: "Model AM-6", product of Atmax, Inc., nozzle discharge slit diameter: 1.1 mm) into a 5 mass% calcium chloride aqueous solution at a liquid conveyance speed of 1.0 mL/min and a spraying gas pressure of 0.3 MPa, to form W/O/W trilayer microcapsules. The W/O/W trilayer microcapsules were filtered and collected using 5A filter paper (product of Advantech Toyo Kaisha, Ltd.), and were rinsed with a 3-fold amount of distilled water. The W/O/W trilayer microcapsules were filtered again with 5A filter paper to collect the W/O/W trilayer microcapsules.

The volume-average particle size of the obtained W/O/W trilayer microcapsules was measured with a laser diffraction/scattering particle size distribution meter (trade name: "SALD-3000", product of Shimadzu Corp.), and the volume-average particle size was found to be 25 µm.

### (Example 2)

The same procedure was carried out as in Example 1, except for using a sodium alginate aqueous solution with a concentration of 0.75 mass% and a viscosity of 70 mPa·s, to obtain a W/O/W dispersion liquid comprising secondary dispersion particles with a volume-average particle size of 3 µm. This W/O/W dispersion liquid was used for the same procedure as in Example 1, and the W/O/W trilayer microcapsules were collected. The volume-average particle size of the obtained W/O/W trilayer microcapsules was 26 µm.

### (Example 3)

The same procedure was carried out as in Example 1, except for using a sodium alginate aqueous solution with a concentration of 0.5 mass% and a viscosity of 15 mPa·s, to obtain a W/O/W dispersion liquid comprising secondary dispersion particles with a volume-average particle size of 8 µm. This W/O/W dispersion liquid was used for the same procedure as in Example 1, and the W/O/W trilayer microcapsules were collected. The volume-average particle size of the obtained W/O/W trilayer microcapsules was 36 µm.

### (Example 4)

The same procedure was carried out as in Example 1, except for using a spray nozzle (trade name: "Model AM-12", product of Atmax, nozzle discharge slit diameter: 1.2 mm) in the spraying step, and the W/O/W trilayer microcapsules were collected. The volume-average particle size of the obtained W/O/W trilayer microcapsules was 89 µm.

### (Example 5)

The same procedure was carried out as in Example 1, except for using a spray nozzle (trade name: "Model AM-25", product of Atmax, nozzle discharge slit diameter: 1.7 mm) in the spraying step, and the W/O/W trilayer microcapsules were collected. The volume-average particle size of the obtained W/O/W trilayer microcapsules was 198 µm.

### (Example 6)

The same procedure was carried out as in Example 4, except for changing the content of the 20 mass% polyphenol aqueous solution to 5.8 g with respect to 14.4 g of vitamin E, and the W/O/W trilayer microcapsules were collected. The volume-average particle size of the obtained W/O/W trilayer microcapsules was 143 µm.

### (Example 7)

The same procedure was carried out as in Example 4, except for changing the content of the 20 mass% polyphenol to 7.2 g with respect to 14.4 g of vitamin E, and the W/O/W trilayer microcapsules were collected. The volume-average particle size of the obtained W/O/W trilayer microcapsules was 135 µm.

### (Example 8)

The same procedure was carried out as in Example 4, except for changing the content of the 20 mass% polyphenol to 11.5 g with respect to 14.4 g of vitamin E, and the W/O/W trilayer microcapsules were collected. The volume-average particle size of the obtained W/O/W trilayer microcapsules was 115 µm.

### (Example 9)

The same procedure was carried out as in Example 4, except for changing the content of the 20 mass% polyphenol to 14.4 g with respect to 14.4 g of vitamin E, and the W/O/W trilayer microcapsules were collected. The volume-average particle size of the obtained W/O/W trilayer microcapsules was 117 µm.

### (Example 10)

The same procedure was carried out as in Example 4, except for changing the spraying gas pressure to 0.1 MPa, and the W/O/W trilayer microcapsules were collected. The volume-average particle size of the obtained W/O/W trilayer microcapsules was 156 µm.

### (Example 11)

The same procedure was carried out as in Example 4, except for changing the spraying gas pressure to 0.5 MPa, and the W/O/W trilayer microcapsules were collected. The volume-average particle size of the obtained W/O/W trilayer microcapsules was 72 µm.

### (Example 12)

The same procedure was carried out as in Example 4, except for changing the calcium ion concentration of the calcium chloride aqueous solution to 0.5 mass%, and the W/O/W trilayer microcapsules were collected. The volume-average particle size of the obtained W/O/W trilayer microcapsules was 97 µm.

### (Example 13)

The same procedure was carried out as in Example 4, except for changing the calcium ion concentration of the calcium chloride aqueous solution to 10 mass%, and the W/O/W trilayer microcapsules were collected. The volume-average particle size of the obtained W/O/W trilayer microcapsules was 90 µm.

### (Example 14)

The same procedure was carried out as in Example 4, except for changing the calcium ion concentration of the calcium chloride aqueous solution to 20 mass%, and the W/O/W trilayer microcapsules were collected. The volume-average particle size of the obtained W/O/W trilayer microcapsules was 124 µm.

### (Example 15)

The same procedure was carried out as in Example 4, except for changing the 5 mass% calcium chloride aqueous solution to a 2.5 mass% calcium sulfate aqueous solution, and the W/O/W trilayer microcapsules were collected. The volume-average particle size of the obtained W/O/W trilayer microcapsules was 133 µm.

### (Example 16)

The same procedure was carried out as in Example 4, except for changing the 5 mass% calcium chloride aqueous solution to a 2.5 mass% calcium lactate aqueous solution, and the W/O/W trilayer microcapsules were collected. The volume-average particle size of the obtained W/O/W trilayer microcapsules was 119 µm.

### (Example 17)

The same procedure was carried out as in Example 4, except for changing the concentration of the sodium alginate aqueous solution to 0.2 mass% and changing the viscosity of the sodium alginate aqueous solution to 5 mPa·s, and the W/O/W trilayer microcapsules were collected. The volume-average particle size of the obtained W/O/W trilayer microcapsules was 114 µm.

### (Example 18)

The same procedure was carried out as in Example 4, except for changing the concentration of the sodium alginate aqueous solution to 1.2 mass% and changing the viscosity of the sodium alginate aqueous solution to 500 mPa·s, and the W/O/W trilayer microcapsules were collected. The volume-average particle size of the obtained W/O/W trilayer microcapsules was 124 µm.

### (Example 19)

The same procedure was carried out as in Example 4, except for changing the concentration of the sodium alginate aqueous solution to 1.5 mass% and changing the viscosity of the sodium alginate aqueous solution to 1000 mPa·s, and the W/O/W trilayer microcapsules were collected. The volume-average particle size of the obtained W/O/W trilayer microcapsules was 112 µm.

### (Example 20)

The same procedure was carried out as in Example 4, except for changing the concentration of the sodium alginate aqueous solution to 2.0 mass% and changing the viscosity of the sodium alginate aqueous solution to 2000 mPa·s, and the W/O/W trilayer microcapsules were collected. The volume-average particle size of the obtained W/O/W trilayer microcapsules was 117 µm.

### (Example 21)

The same procedure was carried out as in Example 4, except for changing the content of the W/O dispersion liquid to 9.3 g with respect to 187.1 g of the sodium alginate aqueous solution, and the W/O/W trilayer microcapsules were collected. The volume-average particle size of the obtained W/O/W trilayer microcapsules was 67 µm.

### (Example 22)

The same procedure was carried out as in Example 4, except for changing the content of the W/O dispersion liquid to 65.4 g with respect to 131.0 g of the sodium alginate aqueous solution, and the W/O/W trilayer microcapsules were collected. The volume-average particle size of the obtained W/O/W trilayer microcapsules was 142 µm.

### (Example 23)

The same procedure was carried out as in Example 4, except for changing the content of the W/O dispersion liquid to 98.2 g with respect to 98.2 g of the sodium alginate aqueous solution, and the W/O/W trilayer microcapsules were collected. The volume-average particle size of the obtained W/O/W trilayer microcapsules was 139 µm.

### (Microscope observation)

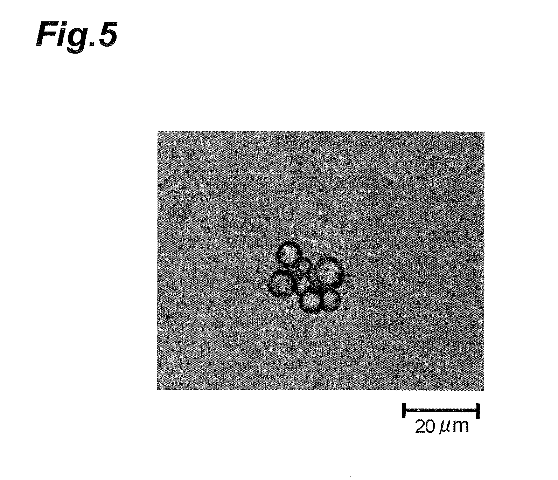

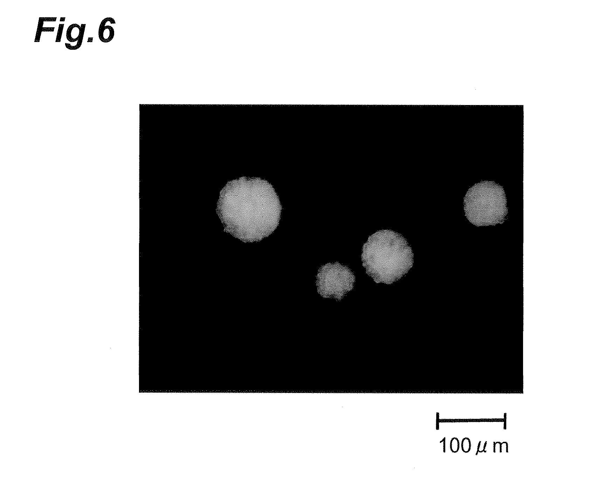

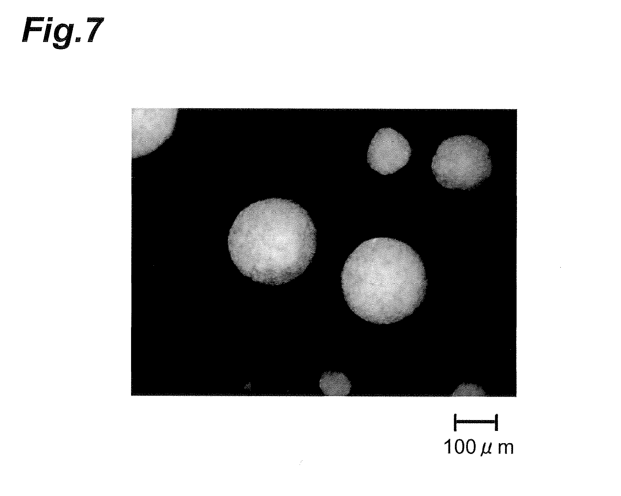

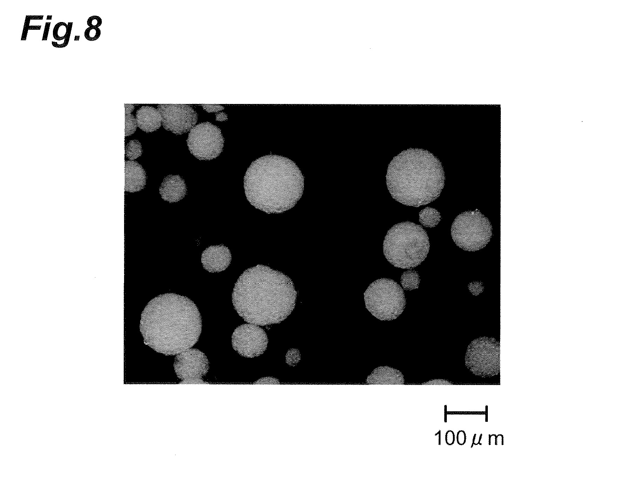

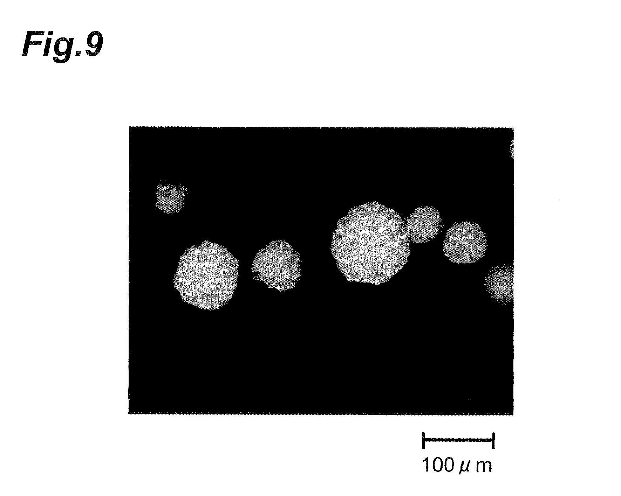

The W/O/W trilayer microcapsules obtained in Example 1 were observed under an optical microscope (trade name: "BX-51-PRF", product of Olympus Corp.). Fig. 5 is an optical microscope photograph of the W/O/W trilayer microcapsules obtained in Example 1. The W/O/W trilayer microcapsules obtained in Example 4, Example 5, Example 13 and Example 19 were observed with a digital microscope (trade name: " Digital Microscope VHX-100F", product of Keyence Corp.). Figs. 6 to 9 are photographs of the W/O/W trilayer microcapsules obtained in Example 4, Example 5, Example 13 and Example 19, respectively.

### (Degree of deformation of microcapsules)

The W/O/W trilayer microcapsules obtained in Examples 1-23 were observed under an optical microscope in the manner described above, and the long diameters and short diameters of the W/O/W trilayer microcapsules were measured for calculation of the degree of deformation. The degree of deformation was calculated for 50 W/O/W trilayer microcapsules, and the average of the 50 degrees of deformation was recorded as the degree of deformation for each example.

### <Measurement of contained ratio of W/O/W trilayer microcapsules>

The W/O/W trilayer microcapsules obtained in Examples 1-23 were dried at 105°C for 6 hours, and the dry mass was measured. Ethanol was then added to the dried microcapsules, and the mixture was stirred at 700 rpm for 12 hours. Next, an ultrasonic wave homogenizer (trade name: "VP-050", product of Tietech Co., Ltd.) was used for 40 minutes of treatment, and the treated microcapsules were crushed. The ethanol solution containing crushed microcapsules was centrifuged at 7000 rpm for 10 minutes, the supernatant was collected, and the absorbance of the supernatant at 285 nm was measured using a spectrophotometer (trade name: " spectrophotometer U-3210", product of Hitachi Instruments Service Co., Ltd.). The polyphenol and vitamin E mass ratios were each determined from the measured absorbance, and the contained ratios of polyphenol and vitamin E in the W/O/W trilayer microcapsules were calculated.

### <W/O/W trilayer microcapsule durability test>

The W/O/W trilayer microcapsules obtained in Examples 1, 3 and 19 were suspended in distilled water, and the suspension was shaken for 1 hour at a speed of 240 rpm using a shaker (trade name: "SA-31", product of Yamato Scientific Co., Ltd.). The same procedure as for measurement of the contained ratio was then carried out, and the contained ratios of polyphenol and vitamin E in the W/O/W trilayer microcapsules were calculated. The durability of the polyphenol-containing layer and vitamin E-containing layer was evaluated based on the residue ratio of polyphenol and vitamin E after shaking, with the contained ratio before shaking as 100%.

Tables 1-4 show the preparation conditions and the results of each measurement (mean particle size, degree of deformation, and contained ratios of polyphenol and vitamin E), for the W/O/W trilayer microcapsules obtained in Examples 1-23. Also, Table 5 shows the results of the durability tests for the W/O/W trilayer microcapsules obtained in Example 1, 3 and 19.

**[Table 1]**

| | | Example 1 | Example 2 | Example 3 | Example 4 | Example 5 |
|---|---|---|---|---|---|---|
| Primary dispersion step | Dispersion conditions | 12000 rpm 5 min/60°C | 12000 rpm 5 min/60°C | 12000 rpm 5 min/60°C | 12000 rpm 5 min/60°C | 12000 rpm 5 min/60°C |
| | Water-soluble substance : fat-soluble substance (mass ratio) | 27.8:100 | 27.8:100 | 27.8:100 | 27.8:100 | 27.8:100 |
| Secondary dispersion step | Dispersion conditions | 8000 rpm 5 min/60°C | 8000 rpm 5 min/60°C | 8000 rpm 5 min/60°C | 8000 rpm 5 min/60°C | 8000 rpm 5 min/60°C |
| | Primary dispersion liquid : Na alginate aqueous solution (mass ratio) | 11.3:100 | 11.3:100 | 11.3:100 | 11.3:100 | 11.3:100 |
| | Na alginate viscosity (mPa·s) | 80 | 70 | 15 | 80 | 80 |
| | Na alginate concentration (mass%) | 1.0 | 0.75 | 0.5 | 1.0 | 1.0 |
| | Mean particle size of secondary dispersion particle (µm) | 3 | 3 | 8 | 6.0 | 6.0 |
| Spraying step | Ca ion-containing solution type | Calcium chloride | Calcium chloride | Calcium chloride | Calcium chloride | Calcium chloride |
| | Concentration of Ca ion-containing solution (mass%) | 5 | 5 | 5 | 5 | 5 |
| | Nozzle discharge slit diameter (mm) | 1.1 | 1.1 | 1.1 | 1.2 | 1.7 |
| | Spraying gas pressure (MPa) | 0.3 | 0.3 | 0.3 | 0.3 | 0.3 |
| | Liquid conveyance speed (mL/min) | 1.0 | 1.0 | 1.0 | 1.0 | 1.0 |
| Mean particle size of microcapsule (µm) | | 25 | 26 | 36 | 89 | 198 |
| Degree of deformation (long diameter/short diameter) | | 1.34 | 1.35 | 1.72 | 1.15 | 1.08 |
| Contained ratio of polyphenol (%) | | 1.5 | 1.2 | 1.0 | 2.4 | 1.6 |
| Contained ratio of vitamin E (%) | | 67.3 | 73.2 | 65.7 | 77.4 | 76.0 |

**[Table 2]**

| | | Example 6 | Example 7 | Example 8 | Example 9 | Example 10 | Example 11 |
|---|---|---|---|---|---|---|---|
| Primary dispersion step | Dispersion conditions | 12000 rpm min/60°C | 12000 rpm 5 min/60°C | 12000 rpm 5 min/60°C | 12000 rpm 5 min/60°C | 12000 rpm 5 min/60°C | 12000 rpm 5 min/60°C |
| | Water-soluble substance : fat-soluble substance (mass ratio) | 40:100 | 50:100 | 80:100 | 100:100 | 27.8:100 | 27.8:100 |
| Secondary dispersion step | Dispersion conditions | 8000 rpm 5 min/60°C | 8000 rpm 5 min/60°C | 8000 rpm 5 min/60°C | 8000 rpm 5 min/60°C | 8000 rpm 5 min/60°C | 8000 rpm 5 min/60°C |
| | Primary dispersion liquid : Na alginate aqueous solution (mass ratio) | 11.3:100 | 11.3:100 | 11.3:100 | 11.3:100 | 11.3:100 | 11.3:100 |
| | Na alginate viscosity (mPa·s) | 80 | 80 | 80 | 80 | 80 | 80 |
| | Na alginate concentration (mass%) | 1.0 | 1.0 | 1.0 | 1.0 | 1.0 | 1.0 |
| | Mean particle size of secondary dispersion particle (µm) | 9.3 | 15.7 | 10.9 | 6.9 | 4.0 | 4.0 |
| Spraying step | Ca ion-containing solution type | Calcium chloride | Calcium chloride | Calcium chloride | Calcium chloride | Calcium chloride | Calcium chloride |
| | Concentration of Ca ion-containing solution (mass%) | 5 | 5 | 5 | 5 | 5 | 5 |
| | Nozzle discharge slit diameter (mm) | 1.2 | 1.2 | 1.2 | 1.2 | 1.2 | 1.2 |
| | Spraying gas pressure (MPa) | 0.3 | 0.3 | 0.3 | 0.3 | 0.1 | 0.5 |
| | Liquid conveyance speed (mL/min) | 1.0 | 1.0 | 1.0 | 1.0 | 1.0 | 1.0 |
| Mean particle size of microcapsule (µm) | | 143 | 135 | 115 | 117 | 156 | 72 |
| Degree of deformation (long diameter/short diameter) | | 1.24 | 1.22 | 1.19 | 1.26 | 1.11 | 1.11 |
| Contained ratio of polyphenol (%) | | 3.7 | 4.7 | 6.6 | 8.0 | 0.5 | 1.7 |
| Contained ratio of vitamin E (%) | | 75.0 | 73.3 | 71.7 | 67.7 | 68.2 | 77.7 |

**[Table 3]**

| | | Example 12 | Example 13 | Example 14 | Example 15 | Example 16 |
|---|---|---|---|---|---|---|
| Primary dispersion step | Dispersion conditions | 12000 rpm 5 min/60°C | 12000 rpm 5 min/60°C | 12000 rpm 5 min/60°C | 12000 rpm 5 min/60°C | 12000 rpm 5 min/60°C |
| | Water-soluble substance : fat-soluble substance (mass ratio) | 27.8:100 | 27.8:100 | 27.8:100 | 27.8:100 | 27.8:100 |
| Secondary dispersion step | Dispersion conditions | 8000 rpm 5 min/60°C | 8000 rpm 5 min/60°C | 8000 rpm 5 min/60°C | 8000 rpm 5 min/60°C | 8000 rpm 5 min/60°C |
| | Primary dispersion liquid : Na alginate aqueous solution (mass ratio) | 11.3:100 | 11.3:100 | 11.3:100 | 11.3:100 | 11.3:100 |
| | Na alginate viscosity (mPa·s) | 80 | 80 | 80 | 80 | 80 |
| | Na alginate concentration (mass%) | 1.0 | 1.0 | 1.0 | 1.0 | 1.0 |
| | Mean particle size of secondary dispersion particle (µm) | 4.0 | 4.0 | 4.0 | 4.0 | 4.0 |
| Spraying step | Ca ion-containing solution type | Calcium chloride | Calcium chloride | Calcium chloride | Calcium sulfate | Calcium lactate |
| | Concentration of Ca ion-containing solution (mass%) | 0.5 | 10 | 20 | 2.5 | 2.5 |
| | Nozzle discharge slit diameter (mm) | 1.2 | 1.2 | 1.2 | 1.2 | 1.2 |
| | Spraying gas pressure (MPa) | 0.3 | 0.3 | 0.3 | 0.3 | 0.3 |
| | Liquid conveyance speed (mL/min) | 1.0 | 1.0 | 1.0 | 1.0 | 1.0 |
| Mean particle size of microcapsule (µm) | | 97 | 90 | 124 | 133 | 119 |
| Degree of deformation (long diameter/short diameter) | | 1.19 | 1.14 | 1.12 | 1.19 | 1.20 |
| Contained ratio of polyphenol (%) | | 1.4 | 1.3 | 1.4 | 0.8 | 1.6 |
| Contained ratio of vitamin E (%) | | 78.7 | 70.1 | 67.6 | 75.6 | 72.1 |

**[Table 4]**

| | | Example 17 | Example 18 | Example 19 | Example 20 | Example 21 | Example 22 | Example 23 |
|---|---|---|---|---|---|---|---|---|
| Primary dispersion step | Dispersion conditions | 12000 rpm 5 min/60°C | 12000 rpm 5 min/60°C | 12000 rpm 5 min/60°C | 12000 rpm 5 min/60°C | 12000 rpm 5 min/60°C | 12000 rpm 5 min/60°C | 12000 rpm 5 min/60°C |
| | Water-soluble substance : fat-soluble substance (mass ratio) | 27.8:100 | 27.8:100 | 27.8:100 | 27.8:100 | 27.8:100 | 27.8:100 | 27.8:100 |
| Secondary dispersion step | Dispersion conditions | 8000 rpm 5 min/60°C | 8000 rpm 5 min/60°C | 8000 rpm 5 min/60°C | 8000 rpm 5 min/60°C | 8000 rpm 5 min/60°C | 8000 rpm 5 min/60°C | 8000 rpm 5 min/60°C |
| | Primary dispersion liquid : Na alginate aqueous solution (mass ratio) | 11.3:100 | 11.3:100 | 11.3:100 | 11.3:100 | 5:100 | 50:100 | 100:100 |
| | Na alginate viscosity (mPa·s) | 5 | 500 | 1000 | 2000 | 80 | 80 | 80 |
| | Na alginate concentration (mass%) | 0.2 | 1.2 | 1.5 | 2.0 | 1.0 | 1.0 | 1.0 |
| | Mean particle size of secondary dispersion particle (µm) | 9.3 | 8.0 | 14.4 | 10.8 | 8.9 | 10.3 | 9.0 |
| Spraying step | Ca ion-containing solution type | Calcium chloride | Calcium chloride | Calcium chloride | Calcium chloride | Calcium chloride | Calcium chloride | Calcium chloride |
| | Concentration of Ca ion-containing solution (mass%) | 5 | 5 | 5 | 5 | 5 | 5 | 5 |
| | Nozzle discharge slit diameter (mm) | 1.2 | 1.2 | 1.2 | 1.2 | 1.2 | 1.2 | 1.2 |
| | Spraying gas pressure (MPa) | 0.3 | 0.3 | 0.3 | 0.3 | 0.3 | 0.3 | 0.3 |
| | Liquid conveyance speed (mL/min) speed | 1.0 | 1.0 | 1.0 | 1.0 | 1.0 | 1.0 | 1.0 |
| Mean particle size of microcapsule (µm) | | 114 | 124 | 112 | 117 | 67 | 142 | 139 |
| Degree of deformation (long diameter/short diameter) | | 1.33 | 1.19 | 1.11 | 1.12 | 1.15 | 1.18 | 1.20 |
| Contained ratio of polyphenol (%) | | 1.7 | 1.5 | 1.2 | 1.4 | 0.5 | 2.8 8 | 2.8 |
| Contained ratio of vitamin E (%) | | 80.1 | 94.1 | 75.6 | 75.0 | 57.9 | 87.7 | 69.2 |

**[Table 5]**

| | Example 1 | Example 3 | Example 19 |
|---|---|---|---|
| Durability of polyphenol-containing layer (%) | 95.5 | 85.5 | 99.8 |
| Durability of vitamin E-containing layers (%) | 97.6 | 90.7 | 100 |

Based on Tables 1-4, the W/O/W trilayer microcapsules obtained in Examples 1-23 were confirmed to be microcapsules with small mean particle sizes, low degree of deformation, and nearly spherical shapes.

### Reference Signs List

1: Water-soluble substance, 3: fat-soluble substance, 5: sodium alginate aqueous solution, 7: nozzle, 9: calcium ion-containing solution, 10: primary dispersion particle, 15: primary dispersion liquid, 20: secondary dispersion particle, 25: secondary dispersion liquid, 30: calcium alginate gel, 100: microcapsule.

## Claims

1. A method of producing microcapsules, comprising:
a primary dispersion step of mixing a water-soluble substance and a fat-soluble substance to obtain a primary dispersion having the water-soluble substance dispersed in the fat-soluble substance;
a secondary dispersion step of mixing the primary dispersion and a sodium alginate aqueous solution to obtain a secondary dispersion having the primary dispersion dispersed in the sodium alginate aqueous solution; and
a spraying step of spraying the secondary dispersion for contact with a calcium ion-containing solution to form a calcium alginate gel and to obtain microcapsules having the primary dispersion dispersed in the calcium alginate gel.

2. A method of producing microcapsules, comprising a step of obtaining microcapsules having a primary dispersion dispersed in a calcium alginate gel by contacting droplets of a secondary dispersion liquid with a calcium ion-containing solution to form the calcium alginate gel,
wherein the primary dispersion has a water-soluble substance dispersed in a fat-soluble substance, and the secondary dispersion liquid comprises the primary dispersion dispersed in a sodium alginate aqueous solution.

3. The method of producing microcapsules according to claim 1 or 2, wherein the water-soluble substance dispersed in the primary dispersion is a water-soluble substance dispersing a fat-soluble substance that is the same or different from the fat-soluble substance (a water-soluble substance that is the same or different from the water-soluble substance may be dispersed in the fat-soluble substance, or dispersion of either a fat-soluble substance or a water-soluble substance in the other may be repeated several times more).

4. The method of producing microcapsules according to any one of claims 1 to 3, wherein a viscosity of the sodium alginate aqueous solution at 25°C is 5-2000 mPa·s.

5. The method of producing microcapsules according to any one of claims 1 to 4, wherein the calcium ion-containing solution is a calcium chloride aqueous solution, a calcium lactate aqueous solution or a calcium sulfate aqueous solution.

6. Microcapsules obtainable by the method of producing microcapsules according to any one of claims 1 to 5.

7. Microcapsules comprising a calcium alginate gel and having a mean particle size of less than 200 µm, wherein a fat-soluble substance is dispersed in the calcium alginate gel and a water-soluble substance is dispersed in the fat-soluble substance.

8. The microcapsules according to claim 7, wherein
the water-soluble substance is a water-soluble substance dispersing a fat-soluble substance that is the same or different from the fat-soluble substance (a water-soluble substance that is the same or different from the water-soluble substance may be dispersed in the fat-soluble substance, or dispersion of either a fat-soluble substance or a water-soluble substance in the other may be repeated several times more).

9. The microcapsules according to claim 7 or 8, wherein a degree of deformation is less than 1.8.

10. A food or beverage containing the microcapsules according to any one of claims 6 to 9.
